# EUROPEAN PATENT APPLICATION

(11) **EP 4 843 518 A1**
(43) Date of publication of application: **25.11.2026**
(21) Application number: 23853693.2
(22) Date of filing: 22.12.2023
(51) Int. Cl.: A61B 17/17

(54) **SURGICAL GUIDE FOR PLACEMENT OF A PEDICLE SCREW IN REVISION SURGERY AND METHOD FOR PRODUCING SAID SURGICAL GUIDE**

(71) Applicant: Digital Anatomics, S.L., 28919 Leganés (ES)
(72) Inventor: REYERO HUERGA, Alejandro, 24004 León (León) (ES); MOSTAZA SAAVEDRA, Antonio Luis, 24008 León (León) (ES)
(74) Representative: San Martin Alarcia, Esther
(86) International application number: PCT/ES2023/070778
(87) International publication number: WO 2025/133416

(57) **Abstract**

The invention discloses a surgical guide (1) comprising: a first body (11); a second body (12) having a guide orifice (121) for guiding the drill bit (B); and a bridge (13) connecting the first body (11) to the second body (12). The first and second surfaces (111, 131) are in contact respectively with a fixed pedicle screw (TF) and the first vertebra (V1). The first surface (111) is provided on the first body (11) and the second surface (131) is provided on either the second body (12) or the bridge (13). Furthermore, said first and second surfaces (111, 131) are obtained by means of additive manufacturing from medical images of the assembly of the first vertebra (V1), a second vertebra (V2) to which said preexisting fixed pedicle screw (TF) is fixed, and the preexisting fixed pedicle screw (TF) itself.

## Description

### OBJECT OF THE INVENTION

The present invention belongs to the field of medicine, and more particularly to the field of spine surgery revision.

A first object of the present invention is a surgical guide particularly designed for spine revision surgery where an incorrectly positioned pedicular screw needs to be extracted and replaced by a new screw.

A second object of the present invention is a method for producing said surgical guide.

### PRIOR ART

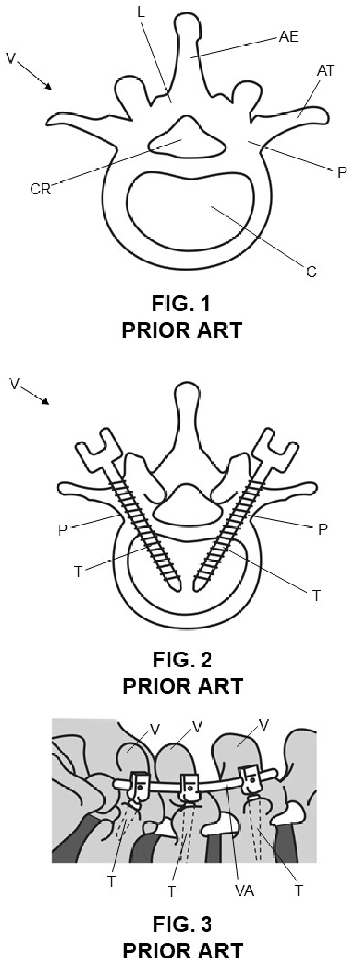

The spine is formed by a number of vertebrae connected ones to the others in an articulated manner. As shown in Fig. 1, a vertebra (V) mainly comprises a vertebral body (C) from which a number of osseous protrusions emerge, such as a central spinous process (AE) and two lateral transverse processes (AT). The spinous process (AE) and the transverse processes (AT) are connected by means of a lamina (L) which, in turn, is connected to the body (C) by means of pedicles (P). Between the lamina (L) and the body (C) there is a cavity known as vertebral foramen (CR) through which the spinal cord passes.

Spine arthrodesis is a well-known technique suitable for certain pathologies mainly consisting of immobilising at least two vertebrae by means of a rigid connection. Thereto, use of so called pedicular screws is known. As shown in Fig. 2, a pedicular screw (T) is a screw that is introduced into the vertebra in a particular direction passing through the pedicle (P). Pedicular screws (T) have a particularly designed head allowing the connection between screws (T) by means of a rod (VA) or the like, thereby achieving the abovementioned immobilisation. Fig. 3 schematically shows three pedicular screws (T) rigidly connected ones with the others by means of the rod (VA).

In this context, it is extremely important that the pedicular screws be introduced into the vertebra through the pedicle along a specific previously-calculated trajectory. Otherwise, problems such as nervous or vascular damage, infection, bone fracture and failure of the immobilisation may arise.

To ensure that the pedicular screws are introduced with the desired trajectory, the use of guides designed for the particular anatomy of each patient is currently known. These guides comprise vertebra support areas having an anatomical shape that is complementary with the anatomical shape of the vertebra they are going to abut against. Thereto, before the surgical procedure medical images of the osseous structures of the patient are acquired and, by means of additive manufacturing, personalized guides fitting perfectly against a surface of the vertebra of the patient are made. This feature enables the guide to be very precisely placed in a particular position onto the vertebra of the patient. The guides also have an orifice oriented in the desired trajectory by means of which the instrument (e.g. a drill) is introduced for drilling a hole in the vertebra through which the screw is subsequently introduced, thereby ensuring a correct placement.

Sometimes, when no guide is used, or even despite its use, positioning errors may arise causing a pedicular screw to be incorrectly positioned. In these cases, a second surgical procedure commonly known as "*surgery revision*" is needed where the incorrectly positioned screw is extracted and replaced by another screw. During the procedure, it is extremely important to ensure that the new screw follows the planned trajectory. This is particularly complicated, given the fact that there is already an orifice in the vertebra that the new screw will tend to follow during the introduction.

There is currently no guide device specifically designed for this purpose.

### DESCRIPTION OF THE INVENTION

The inventors of the present invention have developed a surgical guide particularly intended for placing pedicular screws in revision surgery.

Indeed, in addition to the screw to be replaced, in revision surgery there is normally at least another preexisting pedicular screw inserted in the vertebra where the pedicular screw is going to be replaced, or else in an adjacent vertebra. The solution proposed in the present application is a surgical guide having a surface configured to fit onto the head of said preexisting screw.

The main advantage of this new surgical guide is that the preexisting screw provides a much more firm and stable support than the natural anatomical surfaces of the vertebra conventional guides abut against. A first reason is that, unlike the preexisting pedicular screw, the anatomical surfaces are usually covered with tissues that negatively affect the stability of the support. A second reason is connected to the fact that the shape of a pedicular screw head is specifically designed to allow for a firm and non-slidable coupling. Consequently, the proposed surgical guide does not need a large number of additional supports points, or else support points having a large surface. Normally only one small additional support point besides that provided by the preexisting screw suffices.

### First aspect: surgical guide

A first aspect of the present invention is directed to a surgical guide for the placement of a pedicular screw in revision surgery. The proposed guide is configured to guide a drill for making a new hole in a first vertebra for a new pedicular screw. The surgical screw of the invention mainly comprises the following elements: a first body; a second body having a guide orifice for the drill; and a bridge connecting the first body with the second body. These elements are normally made in a single part, although having separated elements subsequently joined ones to the others is also possible.

Until this point, the features of the surgical guide of the invention are known in the art. However, the guide of the invention differs therefrom by comprising a first and a second surfaces configured to contact respectively with a fixed preexisting pedicular screw and with the first vertebra. The first surface configured to couple to a fixed preexisting pedicular screw is comprised in the first body, while the second surface configured to fit onto an anatomical surface of the first vertebra is comprised in one of the second body and the bridge.

The first surface and the second surface are obtained by means of additive manufacturing from medical images of the group formed by the first vertebra, a second vertebra said fixed preexisting pedicular screw is fixed to, the first fixed preexisting pedicular screw itself and, in case it exists, a rod connected to the preexisting fixed pedicular screw and, possibly, to other fixed preexisting pedicular screws. As in the prior art, the medical images can be obtained by means of a number of technologies, as well as combinations between them, the most important being Computerized Axial Tomography (CAT) and Nuclear Magnetic Resonance (MNR).

In a particularly preferred embodiment of the invention, the first surface may comprise a first cavity portion configured to fit onto the head of a fixed preexisting pedicular screw. This first cavity portion is the negative of the shape of the head of the preexisting pedicular screw. The head of this type of pedicular screw usually has a prismatic shape with a circular, square, rectangular or the like cross-section. Therefore, according to another particularly preferred embodiment of the invention, the first cavity portion may have a prismatic shape with a circular, square or rectangular cross-section.

As mentioned earlier in this document, the fixed preexisting pedicular screw the first surface of the surgical guide is fixed to may be connected to a rod connecting said fixed preexisting pedicular screw with other preexisting pedicular screws fixed to other vertebrae for implementing the arthrodesis. Therefore, in another preferred embodiment of the invention, the second surface of the surgical guide comprises, in addition to the first cavity portion, a second cavity portion configured to fit onto said rod connected to the fixed preexisting pedicular screw. Since the rod usually is shaped as a straight bar, the second cavity portion is preferably shaped as a straight groove. This second cavity portion shaped as a straight groove will normally cross the first cavity portion shaped as a prism, thereby ensuring a firm and stable coupling between the first body of the surgical guide and the group formed by the preexisting pedicular screw and said rod.

The bottom of the first and second cavity portions of the first surface may be flat, whereby the first body fits onto the fixed preexisting pedicular screw and, if applicable, the rod, only by contact with the lateral walls of said portions. Alternatively, in preferred embodiments of the invention, also the bottom of said first and second cavity portions may have the shape of the negative of the screw head and, if applicable, the rod. That is, in this case the bottom of the first cavity portion may have a central protrusion with a shape that engages the orifice in the screw head, for example an hexagonal shape (to engage an orifice for an Allen screwdriver), a star shape (to engage an orifice for a star bit screwdriver), a hexalobular shape (to engage an orifice for a Torx screwdriver), or the like. On the other hand, the bottom of the second cavity portion, if it exists, may be shaped as a cylindrical angular section.

On the other hand, according to another preferred embodiment of the invention, the second surface may be provided at the end of a leg protruding from the bridge. The position and specific configuration of this leg may vary depending on the particular geometrical configuration of each application, although preferably the second surface provided at its end is configured to abut against the lamina of the first vertebra in a position adjacent the area where the new hole is going to be drilled. Since the new hole in the first vertebra is going to be drilled at and end of the lamina joining the pedicle, preferably the leg abuts against an area of the lamina near the base of the spinous process. In short, the second surface preferably has an anatomical shape corresponding to the negative of the portion of lamina of the first vertebra adjacent the area where the new hole is going to be drilled.

In another preferred embodiment of the invention alternative to the above, the second surface may be provided at a base of the second body. In this case, the second surface would be configured to abut against the area of the lamina of the first vertebra where the new hole is going to be drilled. That is, unlike in the prior configuration where the leg abuts close to the base of the spinous process of the first vertebra, that is, in a position separated from the new hole, in this case the second surface abuts against an area surrounding the position where the new hole is going to be drilled. Therefore, in a particularly preferred embodiment of the invention, the second surface has an anatomical shape corresponding to the negative of the portion of lamina of the first vertebra where the new hole is going to be drilled. For example, the guide orifice may be provided at the centre of the second surface provided at the base of the second body. The second surface may be, for example, circular and it may encompass the whole base of the second body.

The surgical guide of the invention may have additional surfaces configured to contact a vertebra, preexisting pedicular screw or connection rod between existing pedicular screws. However, thanks to the firmness and stability provided by the surgical guide fitting onto the existing pedicular screw, only the two contact points mentioned in the above paragraphs are necessary. Thus, according to another particularly preferred embodiment of the present invention, the guide is configured to contact anyone between the first vertebra, the second vertebra, the fixed preexisting pedicular screw itself and the rod only through the firs and second surface, with no additional contact points. More specifically, the guide of the invention does not comprise any surface configured to contact the spinous process of the first vertebra. Avoiding contact with the spinous process is advantageous because the spinous process is usually surrounded by tissues preventing a firm and stable support.

The guide of the invention may be configured mainly in two alternative manners. According to a first configuration, the first surface of the guide fits onto a preexisting pedicular screw that is fixed to the same vertebra where the first hole is going to be drilled. Therefore, in this case the first vertebra and the second vertebra are the same vertebra. The preexisting pedicular screw is necessarily provided in a position of the vertebra that is approximately symmetric with that where the new hole is going to be drilled. Therefore, the bridge connecting the two bodies may somehow pass from one side of the vertebral spinous process to the other. This may be implemented, for example, by designing a bridge having a hole or cavity intended to surround, with no contact, the spinous process when the guide is fixed in its natural position. Alternatively, if necessary, it is possible to surgically remove the spinous process to allow for the installation of the guide.

Alternatively, in a second configuration alternative to the first one disclosed above, the first surface of the guide is fixed to a preexisting pedicular screw that is fixed to a different vertebra from that where the new hole is going to be drilled. That is, the new hole is drilled in a first vertebra, but the preexisting pedicular screw the guide is fitted to is placed in a second vertebra different from the first vertebra. Preferably, the first vertebra and the second vertebra are adjacent one to the other. Normally, the first body is fitted to a pedicular screw provided in an adjacent vertebra at the same side of the spinous process as that where the new hole is going to be drilled. Therefore, in this case there are no important protrusions between the preexisting screw the first body is fitted to and the position of the second body where the new hole is going to be drilled. This entails that the bridge connecting both bodies does not need to have any particular shape as the previous configuration.

### Second aspect: production method

A second aspect of the invention is directed to a method for producing a surgical guide for installing a pedicular screw in revision surgery. Again, the surgical guide is configured to guide a drill for drilling a hole in a first vertebra for a new pedicular screw. The method comprises the following steps:
1. Acquiring medical images of a group formed by the first vertebra, a second vertebra to which the fixed preexisting pedicular screw is fixed, and the fixed preexisting pedicular screw itself.
2. Then, making the surgical guide. The surgical guide comprises a first body, a second body having a guide orifice for the drill, and a bridge connecting the first body with the second body. Said surgical guide further comprises first and second surfaces configured to contact respectively with a fixed preexisting pedicular screw and with the first vertebra. The first surface configured to fit onto a fixed preexisting pedicular screw is comprised in the first body. The second surface configured to fit onto an anatomical surface of the first vertebra is comprised in one between the second body and the bridge. In addition, the first surface and the second surface are obtained by means of additive manufacturing from the medical images acquired in the previous step. Even more preferably, the whole surgical guide is obtained by additive manufacturing.

### BRIEF DESCRIPTION OF THE DRAWINGS

Fig. 1 is a schematic view of a vertebra showing the anatomic component parts thereof.
Fig. 2 shows a schematic view of the position of two pedicular screws fixed to a vertebra.
Fig. 3 shows a schematic view of a number of pedicular screws anchored to several vertebrae and interconnected by means of a rod to ensure the arthrodesis.
Fig. 4 shows a schematic perspective view of a surgical guide according to the present invention designed to be coupled to a pedicular screw anchored to the same vertebra where the new hole is going to be drilled.
Fig. 5 shows another schematic perspective view of the surgical guide shown in Fig. 4.
Figs. 6a-6e shows a sequence of schematic views of the process for replacing a faulty pedicular screw using a surgical guide similar to that shown in Figs. 4 and 5.

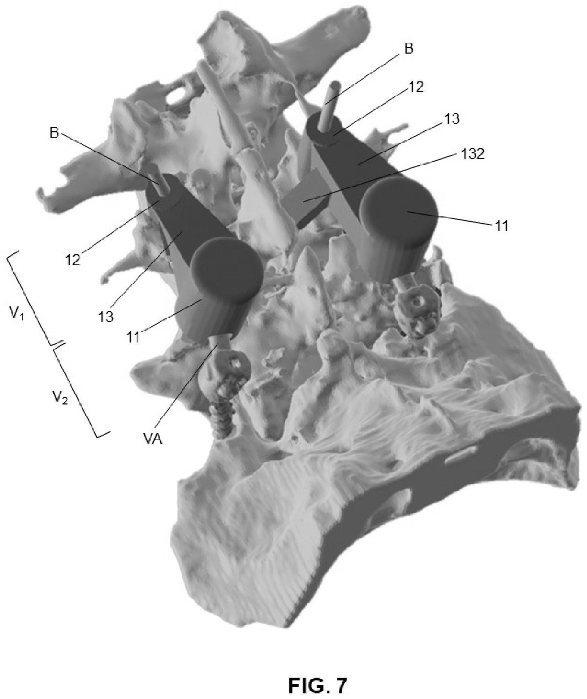

Fig. 7 shows a schematic perspective view of a surgical guide according to the present invention designed to be fixed to a pedicular screw anchored to a vertebra that is adjacent to that where the new hole is going to be drilled.

### PREFERRED EMBODIMENT OF THE INVENTION

Examples of surgical guides (1) according to the present invention are hereon disclosed with reference to the attached drawings. Note that the shapes shown in these figures are simplified, and they therefore do not necessarily correspond exactly to the real shapes of an operative surgical guide.

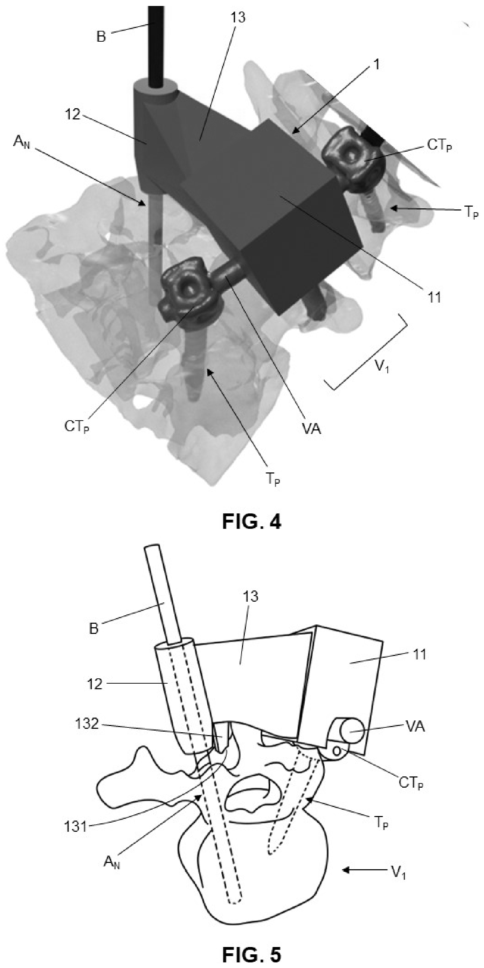

Figs. 4 and 5 show simplified perspective views of a surgical guide (1) designed to abut onto an existing pedicular screw (T_{P}) that is fixed to the same vertebra (V₁) where the new hole(A_{N}) is going to be drilled. That is, according to the notation used in this document, the first vertebra and the second vertebra are one and the same, referred herein simply as "*vertebra (V₁)*". As shown, the surgical guide (1) has a first body (11), a second body (12), and a bridge (13) connecting the first body (11) with the second body (12). In this example, the surgical guide (1) is formed as a single part obtained by means of additive manufacturing in the manner disclosed further down in the present document.

The first body (11) has, at is base oriented towards the vertebra (V₁), a first surface (111) having a prismatic first cavity portion with the same cross-section as the head (CT_{P}) of the preexisting pedicular screw (T_{P}) it is fixed to. Aa shown, the preexisting pedicular screws (T_{P}) have a head (CT_{P}) having an essentially rectangular transverse contour with rounded short sides. Therefore, although not shown in Figs. 4 and 5, the first cavity portion of the first surface (111) has the shape of a rectangular prism with an essentially rectangular cross-section having rounded short sides.

Furthermore, the preexisting pedicular screw (T_{P}) which the first body (11) of the surgical guide (1) is coupled to is connected to a rod (VA) that joins it to two pedicular screws (T_{P}) fixed to vertebrae adjacent to the vertebra (V₁) in question. Therefore, the first surface (111) of the first body (11) of the surgical guide (1) of the present example comprises additionally a second cavity portion shaped as an essentially straight groove (its contour is shown in Fig. 5). This straight groove crosses the rectangular cross-section prism having rounded short sides in perpendicular to the long sides and centred with respect thereof. Thus, the group formed by the head (CT_{P}) of the preexisting pedicular screw (T_{P}) and a portion of the rod (VA) fixed thereto fit perfectly into the first surface (111). Thus, when the body (11) of the surgical guide (1) is placed such that the first surface (111) fits onto the group formed by the screw (T_{P}) plus the section of rod (VA), the guide (1) as a whole is fixed in an exceptionally firm and stable manner.

Although not shown in the figures, the bottom of the first cavity portion may be configured to coupled to the upper face of the preexisting pedicular screw (T_{P}), that is, the face of the head (CT_{P}) of the preexisting pedicular screw (T_{P}) opposite the vertebra (V₁). In that case, the bottom of the first cavity portion would have an hexalobular protrusion to engage the corresponding cavity in the upper face of the head (CT_{P}) of the preexisting pedicular screw (T_{P}). The same could apply to the bottom of the second cavity portion, although in this case the shape would be that of a cylindrical angular section.

On the other hand, the second body (12) and the bridge (13) connecting the first body (11) with the second body (12) both have in Figs. 4 and 5 a schematic shape. Naturally, as is known in the art, the second body (12) has an orifice (121) serving as a guide for a drill (B) for drilling the new hole (A_{N}). In any case, the most important feature is that, when the surgical guide (1) is fixed to the vertebra (V₁), a leg (132) protrudes from the bridge (13) and contacts an area of the lamina (L) that is near the base of the spinous process (AE). This contact point is close to the place where the new hole (A_{N}) will be drilled. The second surface (131) of the end of the leg (132) contacting the lamina (L) has an anatomical shape corresponding to the negative of the shape of the lamina (L) at that point.

The shape of the first surface (111) in the first body (11) and the shape of the second surface (131) of the leg (132) are obtained from medical images of the group formed by the vertebra (V₁), the existing pedicular screw (T_{P}) and the rod (VA) connected to said preexisting pedicular screw (T_{P}). These medical images could be obtained, for example, by NMR or CAT. Subsequently, the surgical guide (1) is made by means of additive manufacturing technologies in a customized manner for the patient in question, using therefor the images acquired previously. The information of the images is used to design the form of the first surface (111) and the second surface (131), as well as the dimensions of the elements making up the surgical guide (1). Normally, not only these surfaces (111, 131), but all of the surgical guide (1) are made by additive manufacturing.

Figs. 5a-5e schematically show how to carry out a revision surgery where a faulty pedicular screw (T_{D}) is replaced by a new pedicular screw (T_{N}).

Firstly, as shown in Fig. 5a, the faulty pedicular screw (T_{D}) is extracted, thereby leaving in the vertebra (V₁) a faulty hole (A_{D}) whose trajectory does not match the desired trajectory. In this figure, also a preexisting pedicular screw (T_{P}) is shown fixed to the vertebra (V₁) at the opposite side thereof, that is, at the other side of the spinous process (AE).

Then, as shown in Fig. 5b, the surgical guide (1) is anchored to the preexisting pedicular screw (T_{P}) by fitting the head (CT_{P}) of said pedicular screw (T_{P}) into the first surface (111) of the first body (11). At the same time, the second surface (131) provided at the end of the leg (132) protruding from the bridge (13) abuts against the area of the lamina (L) provided near the position of the faulty hole (A_{D}), specifically at an area adjacent the base of the spinous process (AE) of the vertebra (V₁). The first and second surfaces (111, 131) are the only two support points of the surgical guide (1), and they are enough to position it with respect to the vertebra (V₁) in a sufficiently rigid and stable manner. In that position, the orifice (121) of the second body (12) is positioned and oriented such that its axis (E₁₂₁) follows the desired trajectory for the new hole (A_{N}). The figure shows that this axis (E₁₂₁) is not aligned with respect to the axis (E_{AD}) of the faulty hole (A_{D}).

In Fig. 5c, a drill (B) connected to a suitable apparatus for carrying out the new hole (A_{N}) is used. The drill (B) is passed through the orifice (121) of the second body (12), thereby ensuring that the new hole (A_{N}) follows exactly the desired trajectory.

Fig. 5d shows a situation where the new hole (A_{N}) is finished and the drill (B) is withdrawn.

In Fig. 5e, the new hole (A_{N}) has already been used for introducing a new pedicular screw (T_{N}) following the desired trajectory.

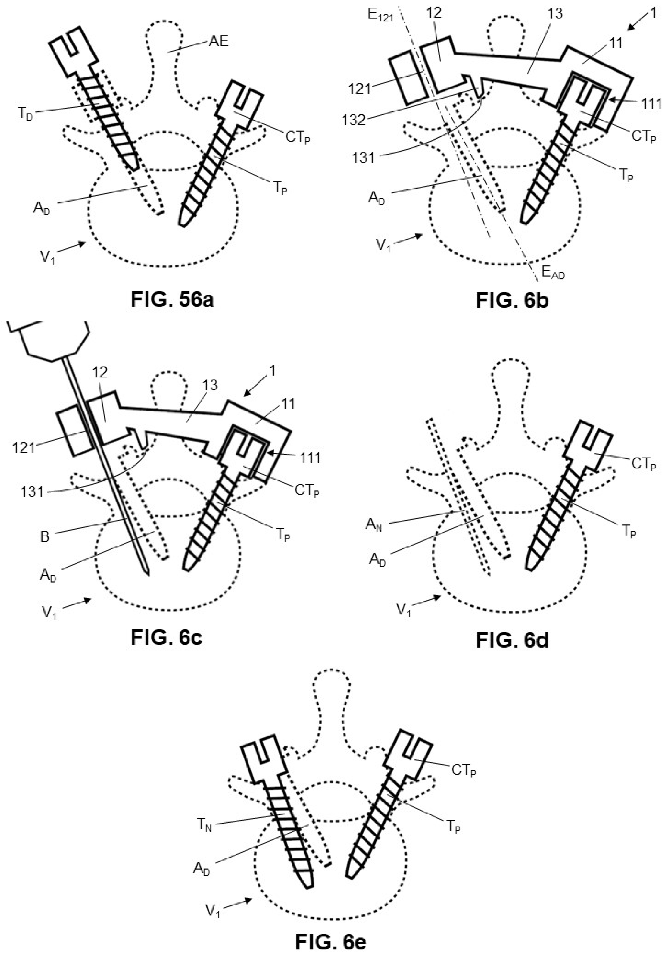

Finally, Fig. 6 shows another configuration where the surgical guide (1) is designed to be coupled with a preexisting pedicular screw (T_{P}) that is fixed to a different vertebra (V₂) than the vertebra (V₁) where the new hole (A_{N}) is going to be drilled. Further, the preexisting pedicular screw (T_{P}) is situated at the same side of the spinous process (AE) as the new hole (A_{N}) that is going to be drilled. Again, the surgical guide (1) has a first body (11), a second body (12), and a bridge (13) connecting the first body (11) with the second body (12). These elements have essentially the same characteristics as those disclosed with respect to the previous configuration except for certain differences.

First, the bridge (13) does not have to have any special configuration here to surpass the spinous process (AE) since, as the two contact points are located at the same side thereof, the plane containing the spinous process (AE) is not crossed. On the other hand, in this configuration the bridge (13) is parallel to a rod (VA) connecting several preexisting pedicular screws (T_{P}), and it has an lower cavity shaped as a groove where said rod (VA) fits.

## Claims

1. Surgical guide (1) for installing a pedicular screw in revision surgery, configured for guiding a drill (B) when drilling a hole (A_{N}) in a first vertebra (V₁) for a new pedicular screw (T_{N}), comprising:
a first body (11);
a second body (12) having a guide orifice (121) for guiding the drill (B); and
a bridge (13) connecting the first body (11) with the second body (12), **characterized by** comprising first and second surfaces (111, 131) configured to contact respectively with a fixed preexisting pedicular screw (T_{F}) and the first vertebra (V₁), where:
the first surface (111) configured to be coupled to a fixed preexisting pedicular screw (T_{F}) is comprised in the first body (11); y
the second surface (131) configured to abut onto an anatomical surface of the first vertebra (V₁) is comprised in one between the second body (12) and the bridge (13),
where said first surface (111) and second surface (131) are obtained by means of additive manufacturing from medical images of the group formed by the first vertebra (V₁), a second vertebra (V₂) to which said fixed preexisting pedicular screw (T_{F}) is fixed, and the fixed preexisting pedicular screw (T_{F}) itself.

2. Surgical guide (1) according to claim 1, where the first surface (111) comprises a first cavity portion configured to fit onto a head (CT_{F}) of the fixed preexisting pedicular screw (T_{F}).

3. Surgical guide (1) according to claim 2, where the shape of the first cavity portion is prismatic with a circular, square or rectangular cross-section.

4. Surgical guide (1) according to any of claims 2-3, where the first surface (111) further comprises a second cavity portion configured to fit onto a rod (VA) connected to the fixed preexisting pedicular screw (T_{F}).

5. Surgical guide (1) according to claim 4, where the second cavity portion is shaped as a straight groove.

6. Surgical guide (1) according to any of the previous claims, where the second surface (131) is provided at an end of a leg (132) protruding from the bridge (13).

7. Surgical guide (1) according to claim 6, where the second surface (131) is configured to abut against the lamina (L) of the first vertebra (V₁) in a position adjacent the area where the new hole (A_{N}) is going to be drilled.

8. Surgical guide (1) according to claim 7, where the second surface (131) has an anatomical shape corresponding to the negative of the portion of lamina (L) of the first vertebra (V₁) adjacent the area where the new hole (A_{N}) is going to be drilled.

9. Surgical guide (1) according to any of claims 1-5, where the second surface (131) is provided at a base of the second body (12) and it is configured to abut against the area of the lamina (L) of the first vertebra (V₁) where the new hole (A_{N}) is going to be drilled.

10. Surgical guide (1) according to claim 9, where the second surface (131) has an anatomical shape corresponding to the negative of the portion of lamina (L) of the first vertebra (V₁) where the new hole (A_{N}) is going to be drilled.

11. Surgical guide (1) according to any of the previous claims, configured to contact any between the first vertebra (V₁), the second vertebra (V₂), the fixed preexisting pedicular screw (T_{F}) itself and the rod (VA) only by means of the first and second surfaces (111, 131).

12. Surgical guide (1) according to claim 11, not comprising any surface configured to contact the spinous process (AE) of the first vertebra (V₁).

13. Surgical guide (1) according to any of the previous claims, where the first vertebra (V₁) and the second vertebra (V₂) are the same vertebra.

14. Surgical guide (1) according to any of claims 1-12, where the first vertebra (V₁) and the second vertebra (V₂) are adjacent vertebrae.

15. Surgical guide (1) according to any of the previous claims, made in its entirety by means of additive manufacturing.

16. Method for producing a surgical guide (1) for the installation of a pedicular screw in revision surgery, where the surgical guide (1) is configured to guide a drill (B) for drilling a hole (A_{N}) in a first vertebra (V₁) for a new pedicular screw (T_{N}), **characterized by** comprising the following steps:
- acquiring medical images of a group formed by the first vertebra (V₁), a second vertebra (V₂) to which a fixed preexisting pedicular screw (T_{F}) is fixed, and the fixed preexisting pedicular screw (T_{F}) itself.
- then, producing the surgical guide (1), where the surgical guide (1) comprises a first body (11), a second body (12) having a guide orifice (121) for guiding the drill (B), and a bridge (13) connecting the first body (11) with the second body (12, said surgical guide (1) comprising first and second surfaces (111, 131) configured to contact respectively with a fixed preexisting pedicular screw (TF) and the first vertebra (V1), where the first surface (111) configured to be coupled to a fixed preexisting pedicular screw (TF) is comprised in the first body (11) y the second surface (131) configured to abut onto an anatomical surface of the first vertebra (V1) is comprised in one between the second body (12) and the bridge (13), where said first surface (111) and second surface (131) are obtained by means of additive manufacturing.
